# EUROPEAN PATENT SPECIFICATION

(11) **EP 2 670 758 B1**
(45) Date of publication and mention of the grant of the patent: **28.09.2022**
(21) Application number: 11709667.7
(22) Date of filing: 08.03.2011
(51) Int. Cl.: C07F 7/18

(54) **PROCESS FOR MAKING TRISUBSTITUTED SILYLOXYETHYL TRIFLATES**
VERFAHREN ZUR HERSTELLUNG VON TRISUBSTITUIERTEN SILYLOXYETHYL-TRIFLATEN
PROCÉDÉ POUR LA FABRICATION DE TRIFLATES DE SILYLOXYÉTHYLE TRISUBSTITUÉS

(30) Priority: 04.02.2011 US 201161439830 P
(43) Date of publication of application: 11.12.2013
(60) Divisional application: 22159989.7
(73) Proprietor: Synthon BV, 6545 CM Nijmegen (NL)
(72) Inventor: BENES , Michal, 190 11 Prague 9 (CZ); JANOUSEK, Vladimir, 190 11 Prague 9 (CZ); PÍS , Jaroslav, 190 11 Prague 9 (CZ)
(74) Representative: Mendivil Gil, Maria Dolores
(86) International application number: PCT/EP2011/053439
(87) International publication number: WO 2012/103960

(56) References cited:
- WO-A1-94/09010
- WO-A1-2007/135397
- US-A1- 2003 125 800
- MOENIUS T ET AL: "Tritium labelling of RAD001- a new rapamycin derivative", JOURNAL OF LABELLED COMPOUNDS AND RADIOPHARMACEUTICALS, JOHN WILEY, CHICHESTER, GB, vol. 43, no. 2, 1 January 2000 (2000-01-01), pages 113-120, XP002335458, ISSN: 0362-4803, DOI: DOI:10.1002/(SICI)1099-1344(200002)43:2&LT ,113::AID-JLCR295&GT,3.0.CO,2- cited in the application
- LEVIN V V ET AL: "Synthesis of C6F5-substituted aminoethanols via acetate ion mediated C6F5-group transfer reaction", SYNTHESIS 20060201 DE LNKD- DOI:10.1055/S-2006-926278, no. 3, 1 February 2006 (2006-02-01), pages 489-495, XP002643849, ISSN: 0039-7881
- DATABASE REGISTRY [Online] CHEMICAL ABSTRACTS SERVICE, COLUMBUS, OHIO, US; 12 September 1997 (1997-09-12), XP002643850, Database accession no. 193967-04-3
- R. N. Haszeldine ET AL: "Perfluoroalkyl derivatives of sulphur. Part I. Trifluoromethanesulphonic acid", Journal of the Chemical Society (Resumed), 1 January 1954 (1954-01-01), page 4228, XP055135182, ISSN: 0368-1769, DOI: 10.1039/jr9540004228
- Macdonald Charles L. B. ET AL: "Indium(i) trifluoromethanesulfonate and other soluble salts for univalent indium chemistry", Chemical Communications, no. 2, 1 January 2004 (2004-01-01), page 250, XP055848281, UK ISSN: 1359-7345, DOI: 10.1039/b312983g
- Armarego Wilfred L. F. ET AL: "Purification of Laboratory Chemicals" In: "Purification of Laboratory Chemicals", 1 January 2003 (2003-01-01), XP055850213, pages 499-499,

## Description

### BACKGROUND OF THE INVENTION

Trisubstituted silyloxyethyltrifluoromethane sulfonates (triflates) of the general formula (3) 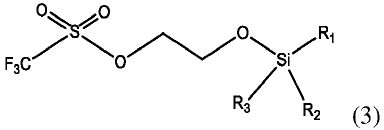
wherein R1,R2,R3 are independently a straight or branched alkyl group of 1 to 10 carbon atoms and/or a phenyl group,
are important reagents useful, i.a., in the synthesis of the pharmaceutically active compound everolimus from rapamycin.

Everolimus, 40-O-(2-hydroxyethyl)-rapamycin of formula (1) is a synthetic derivative of rapamycin (sirolimus) of formula (2), which is produced by a certain bacteria strain and is also pharmaceutically active.

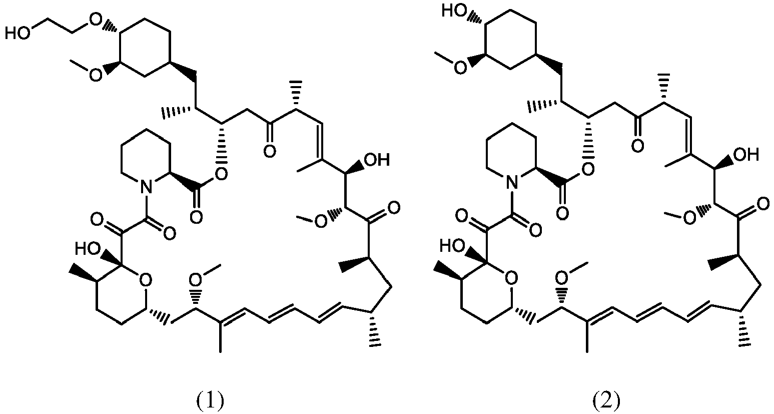

Everolimus is marketed under the brand name Certican for the prevention of rejection episodes following heart and kidney transplantation, and under the brand name Afinitor for treatment of advanced kidney cancer.

Due to its complicated macrolide chemical structure, everolimus is, similarly as the parent rapamycin, an extremely unstable compound. It is sensitive, in particular, towards oxidation, including aerial oxidation. It is also unstable at temperatures higher than 25°C and at alkaline pH.

Everolimus and a process of making it have been disclosed in WO 94/09010 (US 5,665,772, EP 663916). The process principle is shown in the scheme below, wherein the abbreviation RAP-OH has been used as an abbreviation for the rapamycin structure of formula (2) above, L is a leaving group and P is a trisubstituted silyl group serving as a OH-protective group.

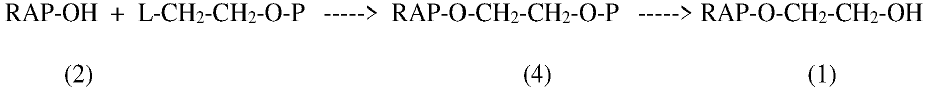

Specifically, the L- group is a trifluoromethanesulfonate (triflate) group and the protective group P- is typically a tert-butyldimethylsilyloxy- group. Accordingly, the known useful reagent within the above general formula (3) for making everolimus from rapamycin is 2-(tert-butyldimethylsilyloxy)ethyl triflate of formula (3A):

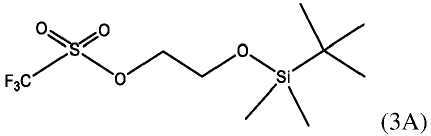

According to a known synthetic procedure disclosed in Example 8 of WO 94/09010 and in Example 1 of US application 2003/0125800, rapamycin (2) reacts in hot toluene and in the presence of 2,6-lutidine with a molar excess of the compound (3A), which is charged in several portions, to form the t-butyldimethylsilyl-protected everolimus (4A). This compound is isolated and deprotected by means of 1N aqueous HCl in methanol. Crude everolimus is then purified by column chromatography. Yields were not reported.

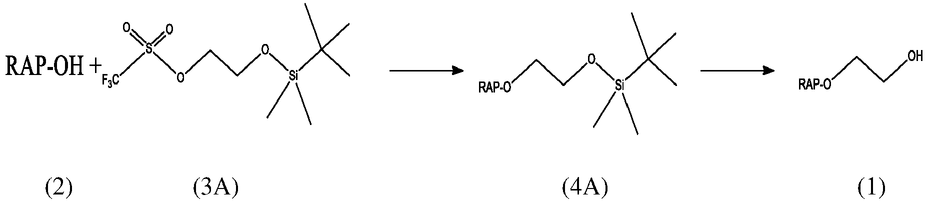

In an article of Moenius et al. (J. Labelled Cpd. Radiopharm. 43, 113-120 (2000)), which used the above process for making C14-labelled and tritiated everolimus, a diphenyl-tert.butylsilyloxy-protective group was used as the alkylation agent of formula (3B).

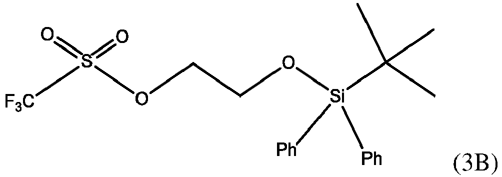

Only 8% yield of the corresponding compound (4B) 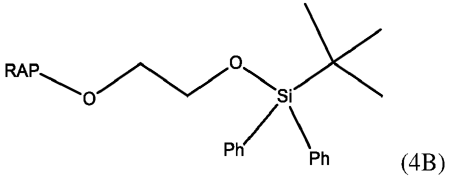 and 21% yield of the compound (1) have been reported.

Little is known about the compounds of the general formula (3) and methods of their preparation. The synthesis of the compound (3A) was disclosed in Example 1 of US application 2003/0125800. It should be noted that specification of the reaction solvent in the key step B of this synthesis was omitted in the disclosure; however, the data about isolation of the product allow for estimation that such solvent is dichloromethane. Similarly also a second article of Moenius et al. (J. Labelled Cpd. Radiopharm.42, 29-41 (1999)) teaches that dichloromethane is the solvent in the reaction.

It appears that the compounds of formula (3) are very reactive, and thus also very unstable compounds. This is reflected by the fact that the yields of the reaction with rapamycine are very low and the compound (3) is charged in high molar extent. Methods how to monitor the reactivity and/or improve the stability of compounds of general formula (3), however, do not exist.

Thus, it would be useful to improve both processes of making compounds of formula (3) and, as well, processes of their application in chemical synthesis.

### BRIEF DESCRIPTION OF THE INVENTION

The present invention relates to an improved process of making the compounds of general formula (3) below. It also relates to an additional process of monitoring their reactivity and stability.

A first aspect of the invention relates to a process of making and/or stabilization of compounds of general formula (3), 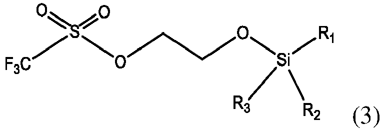 which comprises
a. reacting 2-([trisubstituted]silyloxy)ethanol of general formula (5): 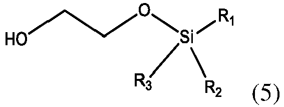 with trifluoromethane sulfonic anhydride, in the presence of a base, wherein the base is selected from N-ethyl-N-isopropyl-2-aminopropane or triisobutylamine,_to form 2-([trisubstituted] silyloxy)ethyl triflate of general formula (3),
   wherein R1,R2,R3 in the compounds of formulas (3) and/or (5) are independently a straight or branched alkyl group of 1 to 10 carbon atoms and/or a phenyl group,
   characterized in that the reaction is carried out in an alkane solvent, having 5 to 12 carbons, such as n-pentane , n-hexane or n-heptane, wherein "alkane" means a straight or branched or cyclic alkane as well as mixtures thereof, including mixtures thereof with an arene, e.g. benzene or toluene.
b. removal of the salt of triflic acid with the base by filtration.

In a specific aspect, the compound of general formula (3) is isolated from the reaction mixture after a step of filtration of the solids out of the reaction mixture.

Preferably, the compound of the general formula (3) is a compound of formula (3A), (3B) or (3C):

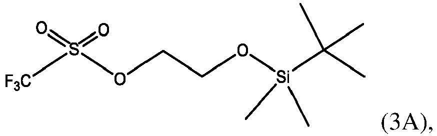

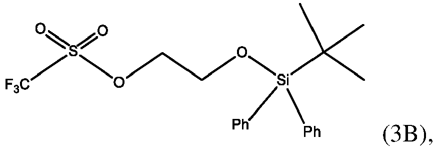

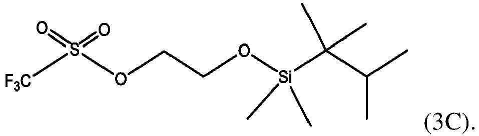

In a yet specific aspect, the compound of the general formula (3) is subjected to a next synthetic step(s) to ultimately form everolimus of formula (1) above.

A specific aspect of the invention relates to a process for the preparation of compounds of general formula (3) substantially free of salts, e.g., trifluoromethane sulfonic acid salts, formed as a side product of reaction of the compound of formula (5) with the base. Removing such salts increases the stability of the compound (3).

A second aspect additionally relates to a process of monitoring the compound of general formula (3), 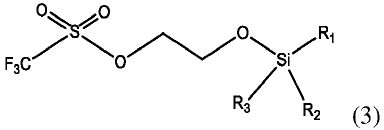
wherein R1,R2,R3 are independently a straight or branched alkyl group of 1 to 10 carbon atoms and/or a phenyl group,
comprising a reaction of the compound of formula (3) with an aromatic secondary amine, typically N-methylaniline, and a detection and/or content determination of the product of the reaction by a suitable analytical technique.

### DETAILED DESCRIPTION OF THE INVENTION

The present invention relates to the process which is a part of a reaction pathway leading to everolimus of formula (1), in which rapamycin of formula (2) is O-alkylated selectively on the position 40 by the compound of general formula (3) 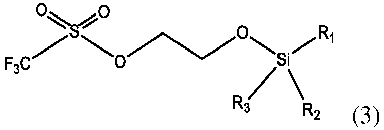 wherein R1, R2, R3 are independently a straight or branched alkyl group of 1 to 10 carbon atoms and/or a phenyl group.

The product of the O-alkylation is then converted to everolimus by removal of the trisubstituted silyl-protective group.

The process is illustrated in the scheme below, wherein the abbreviation RAP-OH has been used as an abbreviation for the rapamycin structure of formula (2) above, L is the triflate leaving group and P is the above defined trisubstituted silyl group serving as a OH-protective group.

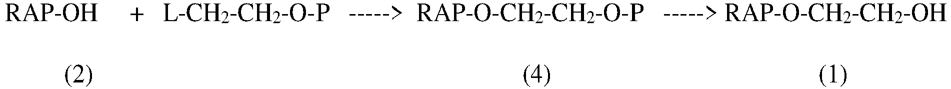

In accordance with the teaching above, proper selection of both the leaving group Land the protective group P- is apparently of utmost importance. For instance, it could be expected that the O-alkylation step on the OH-group attached to carbon 40 of the rapamycin skeleton might be performed under essentially the same conditions as, e.g., on a cyclohexanol. However, common leaving groups for the O-alkylation reaction require relatively harsh reaction conditions so that the alkylation reaction of rapamycin would be accompanied with enormous amounts of side- and decomposition products. Thus, using the extremely reactive trifluoromethane sulfonate (triflate) leaving group is a good option as it may alkylate the OH-group selectively in position 40 under very mild conditions.

However, a reactive compound is also an unstable compound. It has been already discussed above that the yields of the alkylation of rapamycin with the compound of formula (3A) are relatively low and the compound (3A), in a large molar excess, is charged into the reaction mixture in several portions. Both these features indicate that the compound (3A) has been decomposed in a large extent either during storage or at the chosen reaction conditions. In fact, the compound (3A) is actually used *in situ,* i.e. immediately after making it.

Stabilization of the compounds of general formula (3), preferably without a loss of reactivity, would be desirable, however no indication of how to stabilize these compounds has been provided in the prior art.

The compounds of formula (3) may be produced by a process comprising a step of reaction of 2-([trisubstituted]silyloxy)ethanol of formula (5) 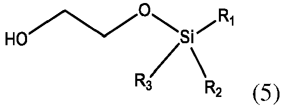 in which Si(R1)(R2)(R3) is as defined above,
with trifluoromethanesulfonic [triflic] acid or triflic acid anhydride in the presence of a base. The process of the invention is defined in the claims. While the prior art documents prefer dichloromethane as the solvent, the present inventors found out that such reaction may advantageously proceed in an alkane solvent, which preferably has 5 to 12 carbons.

The "alkane" in this particular consequence means a straight or branched or cyclic alkane as well as mixtures thereof, including mixtures thereof with an arene, e.g. benzene or toluene.

The "base" in this particular consequence means both an inorganic and organic base and is preferably an amine base.

The key advantage of this alkane solvent is that the second product of the reaction, a salt of triflic acid with the base, is insoluble in this solvent and may be removed by a simple filtration. To the contrary, the dichloromethane solvent dissolves this salt, which must then be removed by a relatively complicated aqueous extraction. It must be noted that the aqueous extraction may also cause formation of side products by partial hydrolysis of the reactive triflate group during the extraction. Thus, apart from its simplicity, the process employing the alkane solvent has the advantage of providing a more pure product.

In more detail, the overall process for making the compound of formula (3) comprises two steps. In a first step, which is not a part of the invention, ethyleneglycol reacts with the corresponding [trisubstuted]silylchloride in an inert solvent or, advantageously, without using a solvent, in the presence of a base, which may be a hydride base or, advantageously, is an amine base. This process provides the compound of general formula (5) above.

In the second step, the compound of formula (5) may react with trifluoromethane sulfonic acid or an activated derivative thereof, e.g. with trifluoromethane sulfonic anhydride (triflic anhydride), in a C5-C12 alkane solvent ( as defined above) , which advantageously may be n-pentane, n-hexane or n-heptane, in the presence of a base, which preferably is an amine base, advantageously a tertiary amine base. The reaction temperature is typically ambient or lower than ambient, advantageously from -10 to -40°C. The formed insoluble triflic salt of the base is removed by filtration and the product is isolated from the filtrate, e.g. by evaporation of the solvent. After isolation and an optional purification, the compound may be obtained as an oily product. The process of the invention is defined in the claims.

The process of the present invention is specifically useful both for making the known compounds of formulas (3A) and (3B) below, and is generally useful also for making various other compounds of the general formula (3). Specifically, one of the most useful compounds in the everolimus synthesis is the 2-(t-hexyldimethylsilyloxy)ethyl triflate compound of formula (3C), which provides everolimus in higher yields and better purity than the known reagents (3A) and/or (3B). This compound may be advantageously made by the above disclosed process and, accordingly, the process of making the compound of formula (3C) by the above synthetic pathway forms a specific aspect of the invention.

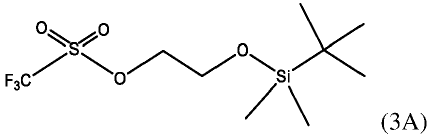

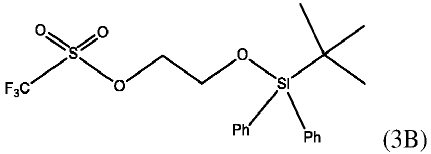

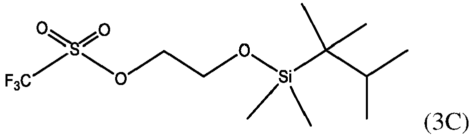

In accordance with the above, a combination of the production step associated with a filtration step may be also used for stabilization of the compound of general formula (3). Thus, the present invention provides a process of stabilization of the compound of general formula (3), which comprises:
a) reacting 2-([trisubstituted]silyloxy)ethanol of general formula (5) with trifluoromethane sulfonic anhydride in the presence of a base, wherein the base is selected from N-ethyl-N-isopropyl-2-aminopropane or triisobutylamine, in an alkane solvent (as defined above) preferably having 5 to 12 carbons, such as n-pentane, n-hexane or n-heptane, to form 2-([trisubstituted] silyloxy)ethyl triflate of general formula (3);
b) removal of formed triflate salts of the base from the reaction mixture by filtration.

The compounds of formula (3) are obtained by the process of the present invention substantially free of salts, particularly salts of triflic acid with the base, which is advantageous as also the presence of such salts in the product decreases the stability of the product. Thus, a compound of general formula (3) substantially free of salts, e.g., trifluoromethane sulfonic acid salts is useful in making everolimus. The "substantially free" preferably means that the product of formula (3) contains less than 10%, preferably less than 5% and in some embodiments less than 1% of the originally formed amount of the salt of triflic acid with the base.

It was found by the present inventors that, because of limited stability, the content and purity of the produced triflate of formula (3) should be advantageously determined before its use in the process of making everolimus. As the most useful triflates (3A) and (3C) are not detectable by analytical methods based on UV detection (because no UV-absorbing group is present in the molecule), conventional analytical methods such as HPLC and TLC fail.

Accordingly, the present invention provides a process for monitoring compounds of the general formula (3) prepared according to the process of the presented invention, i.e. for assaying their content and/or purity, determining their stability and/or detecting their presence. The process is based on a derivatization method , more specifically on the substitution reaction of the triflate group in the compound of general formula (3) with a derivatization agent, which preferably is an aromatic secondary amine, for instance N-methylaniline. The formed tertiary aromatic amine, which now bears a chromophor group, may then be detected and/or its content may be determined by a suitable analytical technique, e.g. by HPLC.

Thus, a sample comprising the compound of formula (3),either in isolated state or in a solvent, is contacted, at essentially ambient temperature, with a solution of the derivatization agent. The derivatization agent should be preferably used in a molar extent, e.g. up to 10 molar extent. The derivatization reaction lasts typically from 10 to 30 minutes. Then the sample is diluted to a suitable concentration and analysed, e.g., by HPLC. In an advantageous mode, a reference material may be prepared from an essentially pure triflate and may be analysed in parallel to obtain a quantitative result.

Details of the analytical process depend on the actual purpose and may be set up by a skilled analyst by routine experimentation.

It should be noted that also related impurities bearing the triflate group may be monitored by the above method, as they are derivatizable the same way.

In a particular aspect, the derivatization process is applied within a process of making the compound of formula (3). The derivatization process may be also used in the process of making everolimus from the compound (3).

In the first step of such process, the compound (3) reacts with rapamycin in an inert solvent and in the presence of a base. The solvent may be an aliphatic, cyclic or aromatic hydrocarbon of 5 to 10 carbon atoms, typically toluene, a chlorinated hydrocarbon of 1 to 6 carbon atoms, typically dichloromethane, an aliphatic or cyclic ether of 4 to 10 carbon atoms and 1 to 3 oxygen atoms, typically dimethoxyethane, and mixtures thereof. The base is typically a highly sterically hindered tertiary amine-type base, as such base is weakly nucleophilic due to sterical hindrance. Examples include tris(2-methylpropyl)amine and N,N-diisopropylethylamine. The molar ratio of the compound (3) to rapamycin preferably does not exceed 5:1, and typically is less than 4:1, in some embodiments less than 3:1.

The reaction is advantageously monitored for the presence of the not yet reacted triflate (3) as it was found that the presence of the unreacted triflate (3) in the reaction mixture should be avoided in the next stage of elaboration of the reaction mixture. For reasons discussed above, the presence/nonpresence of the triflate is advantageously monitored by the above derivatization method based on the reaction of the triflate (3) with a derivatization agent, which preferably is a secondary aromatic amine, typically N-methylaniline.

The improvements of the present invention may also be used in making derivatives of everolimus, particularly those, which use everolimus as the starting material. In a particular aspect, the compounds of formula (3) may be used for protecting or masking the 40-O-(2-hydroxyethyl)-group on the rapamycin ring in a process of making derivatives of everolimus.

The invention is further illustrated by the following examples but they should not be construed as limiting the scope of this invention in any way.

### EXAMPLES

### Example 1: 2-(2,3-dimethylbut-2-yl)dimethylsilyloxy)ethanol

In a 1 L round-bottomed flask ethane-1,2-diol (100.02 g, 1.611 mol) and chloro(2,3-dimethylbutan-2-yl)dimethylsilane (43.40 g, 243 mmol) were mixed to give two liquid colorless non-miscible layers. The flask was placed into a water bath set at 30°C. Pyridine (299 ml, 3.699 mol) was added within approx. 30 sec under stirring, whereby a solution was formed. The reaction mixture was stirred for 1 hr.

After that the mixture was neutralized with 2.00 g of sodium acetate and 12.75 g of sodium carbonate. The mixture was stirred for 20 min at 30°C and left overnight.

Pyridine was distilled off using vacuum (vacuum 50 mbar, bath temperature 50°C-75°C). The residue (yellow suspension) was cooled down and partitioned between water (200 ml) and methyl t-butylether (200 ml). The layers were separated and the organic layer was washed with water (200 ml) and dried over Na₂SO₄ (20 g). The solvents were removed by vacuum distillation (330 to 160 mbar, bath temperature 60°C) to give the crude product as a yellow oil (57.5g). The crude product was further purified by vacuum distillation (vacuum 25 to 2 mbar, batch temperature 90°C to 100°C). Two fractions containing product were collected: fraction 2 (24.10 g, boiling point 50-55°C / 2-3 mbar) and fraction 3 (17.40 g, boiling point 55-60°C / 2-3 mbar). In total 41.2 g (82.9% of the theoretical yield) was obtained.

### Example 2: 2-((2,3-dimethylbut-2-yl)dimethylsilyloxy)ethyl triflate

The reaction was carried out under nitrogen. In a 1 L round-bottomed flask, 2-((2,3-dimethylbut-2-yl)dimethylsilyloxy)ethanol (18.29 g, 89 mmol) and N-ethyl-N-isopropyl-2-aminopropane (12.14 g, 94 mmol) were stirred in n-heptane (366 ml) to give a colorless solution. The solution was cooled down under stirring (bath temperature -30 °C). A 250 mL dropping funnel was charged with a solution of trifluoromethanesulfonic anhydride (25.20 ml, 89 mmol) in n-heptane (183 ml). This solution was added dropwise over 15 min to the reaction mixture (white precipitate started to form immediately) while maintaining the temperature of the reaction mixture between -33°C and -25°C. The reaction mixture was stirred at a temperature between -30°C and -15°C for 120 minutes. The precipitate was filtered off. The filtrate was filtered through silica gel (65 g), the silica gel was washed with heptane (1,000 ml). The combined liquid was evaporated to give the desired product.

The total yield was 26.07 g (86.6% of the theoretical yield).

### Example 3A: Derivatization method for the determination of 2-((2,3-dimethylbut-2-yl)dimethylsilyloxy)ethyl trifluoromethane sulfonate (3C)

### Model sample:

2-((2,3-dimethylbut-2-yl)dimethylsilyloxy)ethanol (232 mg, purity 97.9%, 1.112 mmol) was charged into a reaction flask and pentane (3 ml) was added followed by N-ethyl-N-isopropyl-2-aminopropane (0.203 ml, 1.192 mmol). The reaction mixture was cooled down to 0°C. Trifluoromethanesulfonic anhydride (320 mg, 1.135 mmol) was dissolved in 3 ml of pentane and this solution was added to the stirred reaction mixture over 5 minutes using a syringe while maintaining the temperature at 0 °C. The reaction mixture was stirred at 0°C for 30 min.

### Derivatization:

10 ml of solution of derivatization agent (1M solution of N-methylaniline in acetonitrile:dichloromethane 3:1 v/v) is added to the model sample at once using a pipette and the reaction flask with the mixture is immersed in a water bath (room temperature). The derivatization mixture is stirred at room temperature for 15 min. The content of the reaction flask is transferred to a 50 ml volumetric flask and the volume is made up with acetonitrile. 1.0 ml of this solution is transferred to a 25 ml volumetric flask and the volume is made up with acetonitrile. This sample (10 µl) is injected onto the HPLC and analyzed. The recovery as determined by HPLC is 355mg (95% of the theoretical yield).

### Example 3B: Derivatization method for the determination of 2-(tert-butyldimethylsilyloxy)ethyl trifluoromethanesulfonate (3A)

2-(tert-butyldimethylsilyloxy)ethanol (200 mg, 1.134 mmol) is weighed into a reaction flask and cyclohexane:toluene (1:1) 3 ml is added followed by N-ethyl-N-isopropylpropan-2-amine (0.203 ml, 1.191 mmol) (using a 250 µl syrringe). The reaction mixture is cooled down to 5°C. Trifluoromethanesulfonic anhydride (320 mg, 1.134 mmol) is weighed into a 5 ml vial and cyclohexane:toluene (1:1) 3 ml is added. This solution is added to the reaction mixture over 5 minutes using a syringe while maintaining the temperature at 5°C. The reaction mixture is stirred at 5°Cfor 30 min.

After that 10 ml of solution of derivatization agent (1M solution of N-methylaniline in acetonitrile:dichloromethane 3:1 v/v) is added at once using a pipette and the reaction flask with the reaction mixture is immersed into a water bath (room temperature). The derivatization mixture is stirred at room temperature for 15 min. The derivatization mixture is stirred at room temperature for 15 min. The content of the reaction flask is transferred to a 50 ml volumetric flask and the volume is made up with acetonitrile. 1.0 ml of this solution is transferred to a 25 ml volumetric flask and the volume is made up with acetonitrile. This sample (10 µl) is injected onto the HPLC and analyzed. Recovery determined by HPLC: 297 mg (85 % of the theoretical yield).

### HPLC settings:

| | | |
|---|---|---|
| Analytical column: | Gemini C18 110 A, 150 × 4.6 mm, 5 µm | |
| Guard column: | n.a. | |
| Column temperature | ambient | |
| Flow rate | 1 ml / min | |
| Detection | UV 255 nm | |
| Injection volume | 10 µl | |
| Gradient program: | Time [min] | conc. of B [%] |
| | 0 | 50 |
| | 5 | 55 |
| | 11 | 95 |
| | 18 | 95 |
| | 19 | 50 |
| | 24 | 50 |

Mobile phase A: 20 mM ammonium formiate buffer pH 4.0 (1.26 g of NH₄CO₂H in 1 L of water, pH adjusted to pH=4.0 by (diluted) formic acid).
Mobile phase B: Acetonitrile

### Example 4: Use of derivatization method for testing stability of the triflate (3C)

### a] Stability of the triflate in dichloromethane

To a 10 mL reaction flask 2-((2,3-dimethylbut-2-yl)dimethylsilyloxy)ethanol (200 mg, 0.979 mmol) and triisobutylamine (181 mg, 0.979 mmol) in CH₂Cl₂ (3 ml) were added. The reaction mixture was cooled down to -10°C under nitrogen. In a 5 mL flask, a solution of trifluoromethanesulfonic anhydride (276 mg, 0.979 mmol) in CH₂Cl₂ (3.00 ml) was prepared. This solution was added dropwise to the reaction mixture during 5 min. The reaction mixture (solution) was stirred for 20 min at -10°C. The temperature of the reaction mixture was adjusted to room temperature and the content of triflate was determined by the HPLC method at several time points. The results are presented in the following table (the content of triflate is expressed as % of the theoretical yield corrected for the purity of the starting material 2-((2,3-dimethylbut-2-yl)dimethylsilyloxy)-ethanol).

| Time (hours) | Yield of triflate (% of theor. yield) |
|---|---|
| 0 | 81.89 |
| 1 | 72.52 |
| 2 | 71.50 |
| 4 | 68.48 |
| 6 | 67.68 |

### b) Stability of triflate in pentane

To a reaction flask 2-((2,3-dimethylbut-2-yl)dimethylsilyloxy)ethanol (400 mg, 1.957 mmol) and triisobutylamine (0.474 ml, 1.957 mmol) in 6 ml of pentane were added. The reaction mixture was cooled down to -10°C under nitrogen. In a flask was prepared solution of trifluoromethanesulfonic anhydride ((552 mg, 1.957 mmol) in pentane (6 ml). This solution was added dropwise to the reaction mixture during 5 min. The reaction suspension was stirred at -10°C for 20 min and the solids were filtered off and washed with 5 ml of pentane. The temperature of the filtrate was adjusted to room temperature and the content of triflate was determined by the HPLC method at several time points. The results are presented in the following table (the content of triflate is expressed as % of the theoretical yield corrected for the purity of the starting material 2-((2,3-dimethylbut-2-yl)dimethylsilyloxy)ethanol).

| Time (hours) | Yield of triflate (% of theor. yield) |
|---|---|
| 0 | 89.05 |
| 1 | 89.09 |
| 2 | 89.54 |
| 4 | 90.28 |
| 6 | 90.29 |

### Example 5: 2-(tert-butyldimethylsilyloxy)ethyl trifluoromethanesulfonate

2-(tert-butyldimethylsilyloxy)ethanol (200 mg, 1.134 mmol) was weighed into a reaction flask and cyclohexane 3 ml was added followed by N-ethyl-N-isopropylpropan-2-amine (0.203 ml, 1.191 mmol) (using a 250 ul syrringe). The reaction mixture was cooled down to 10°C. Trifluoromethanesulfonic anhydride (320 mg, 1.134 mmol) was weighed into a 5 ml vial and cyclohexane 3 ml was added. This solution was added to the reaction mixture over 5 minutes using a syringe while maintaining the temperature at 10°C. The reaction mixture was stirred at 10°C for 30 min. The solids were filtered off and washed with 2x 1 ml of cyclohexane. The filtrate was evaporated to dryness to give 323.74 mg of product, assay (HPLC) 87.55%, corresponding to 283.4 mg of 100% material (81% of theoretical yield).

### Example 6: 40-O-[2-((2,3-dimethylbut-2-yl)dimethylsilyloxy)ethyl]rapamycin

In a 100 mL flask, Rapamycin (6 g, 6.56 mmol) was dissolved in dimethoxyethane (4.2 ml) and toluene (24 ml) to give a white suspension and the temperature was raised to 70°C. After 20 min, N,N-diisopropylethylamine (4.56 ml, 27.6 mmol) and 2-((2,3-dimethylbutan-2-yl)dimethylsilyloxy)ethyl trifluoromethanesulfonate (8.83 g, 26.3 mmol) were added in 2 portions with a 2 hr interval at 70°C. The mixture was stirred overnight at room temperature, then diluted with EtOAc (40 ml) and washed with sat. NaHCO₃ (30 ml) and brine (30 ml). The organic layer was dried with Na₂SO₄, filtered and concentrated. The crude product was chromatographed on a silica gel column (EtOAc/heptane 1/1; yield 4.47 g).

### Example 7: 40-O-(2-hydroxyethyl)-rapamycin [everolimus]

In a 100 mL flask, 40-O-[2-((2,3-dimethylbut-2-yl)dimethylsilyloxy)ethyl]rapamycin (4.47 g, 4.06 mmol) was dissolved in methanol (20 ml) to give a colorless solution. At 0°C, 1N aqueous hydrochloric acid (2.0 ml, 2.0 mmol) was added and the mixture was stirred for 90 min. The reaction was followed by TLC (ethyl acetate/n-heptane 3:2) and HPLC. Then 20 ml of saturated aqueous NaHCO₃ were added, followed by 20 ml of brine and 80 ml of ethyl acetate. The phases were separated and the organic layer was washed with saturated aqueous NaCl until pH 6/7. The organic layer was dried by Na₂SO₄, filtered and concentrated to yield 3.3 g of the product.

The invention having been described it will be obvious that the same may be varied in many ways and all such modifications are contemplated as being within the scope of the invention as defined by the following claims.

## Claims

1. A process for making the compound of general formula (3), 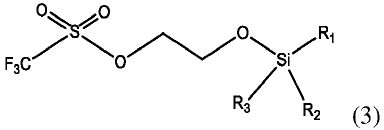 wherein R1,R2,R3 are independently a straight or branched alkyl group of 1 to 10 carbon atoms and/or a phenyl group,
which comprises
a. reacting 2-([trisubstituted]silyloxy)ethanol of general formula (5): 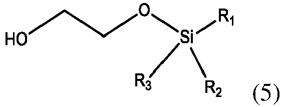
with trifluoromethane sulfonic anhydride, in the presence of a base, to form 2-([trisubstituted] silyloxy)ethyl triflate of general formula (3) and a salt of triflic acid with the base
**characterised in that** the reaction is carried out in an alkane solvent having 5 to 12 carbons, wherein "alkane" means a straight or branched or cyclic alkane as well as mixtures thereof, including mixtures thereof with an arene, e.g. benzene or toluene and wherein the base is selected from N-ethyl-N-isopropyl-2-aminopropane or triisobutylamine
b. removal of the salt of triflic acid with the base by filtration.

2. The process according to claim 1, wherein the alkane is n-pentane, n-hexane or n-heptane.

3. The process according to claims 1-2, wherein the compound (3) is 2-(t-hexyldimethylsilyloxy)ethyl triflate of formula (3C)

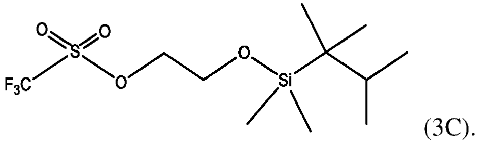

4. The process according to claims 1-3, wherein the compound of formula (3) is isolated from the reaction mixture after removal of solids from said reaction mixture by filtration.

5. The process according to claims 1-4 further comprising a step of subjecting the compound of formula (3) to a next synthetic step(s) to ultimately form everolimus of formula (1).

6. The process according to claim 5, wherein the synthetic step is monitored for the presence of the compound of formula (3) by a derivatization method based on the reaction of the compound (3) with a derivatization agent.

7. The method according to claims 6 wherein the derivatization agent is a secondary aromatic amine.

8. The process according to claims 6-7 wherein the derivatization agent is N-methylaniline.

9. A process of stabilization the compound of general formula (3), 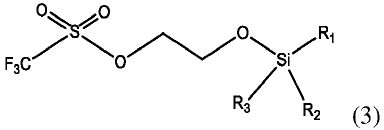 wherein R1,R2,R3 are independently a straight or branched alkyl group of 1 to 10 carbon atoms and/or a phenyl group,
which comprises
a) reacting 2-([trisubstituted]silyloxy)ethanol of general formula (5): 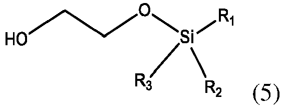 with trifluoromethane sulfonic anhydride, in the presence of a base, wherein the base is selected from N-ethyl-N-isopropyl-2-aminopropane or triisobutylamine,_in an alkane solvent having 5 to 12 carbons, such as n-pentane, n-hexane or n-heptane, wherein "alkane" means a straight or branched or cyclic alkane as well as mixtures thereof, including mixtures thereof with an arene, e.g. benzene or toluene, to form 2-([trisubstituted] silyloxy)ethyl triflate of general formula (3); and
b) removal of the formed triflate salts of the base from the reaction mixture by filtration.

## Patentansprüche

1. Verfahren zum Herstellen der Verbindung der allgemeinen Formel (3), 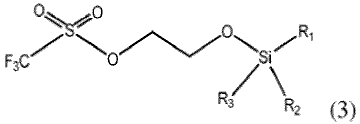 wobei R1, R2, R3 unabhängig eine gerade oder verzweigte Alkylgruppe von 1 bis 10 Kohlenstoffatomen und/oder eine Phenylgruppe sind,
welches
a) Umsetzen von 2-([Trisubstituiertem]silyloxy)ethanol der allgemeinen Formel (5): 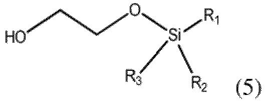
mit Trifluormethansulfonsäureanhydrid in der Gegenwart einer Base umfasst, um 2-([Trisubstituiertes]silyloxy)ethyltriflat der allgemeinen Formel (3) und ein Salz von Triflinsäure mit der Base zu bilden,
**dadurch gekennzeichnet, dass** die Reaktion in einem Alkanlösungsmittel mit 5 bis 12 Kohlenstoffen durchgeführt wird, wobei "Alkan" eine gerades oder verzweigtes oder cyclisches Alkan sowie Gemische davon bedeutet, einschließlich Gemischen davon mit einem Aren, z.B. Benzol oder Toluol, und wobei die Base aus N-Ethyl-N-isopropyl-2-aminopropan oder Triisobutylamin ausgewählt ist
b) Entfernen des Salzes von Triflinsäure mit der Base durch Filtration.

2. Verfahren nach Anspruch 1, wobei das Alkan n-Pentan, n-Hexan oder n-Heptan ist.

3. Verfahren nach Ansprüchen 1-2, wobei die Verbindung (3) 2-(t-Hexyldimethylsilyloxy)ethyltriflat der Formel (3C) ist

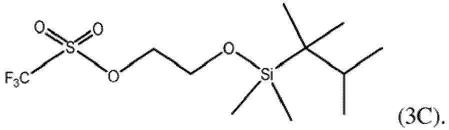

4. Verfahren nach Ansprüchen 1-3, wobei die Verbindung der Formel (3) aus dem Reaktionsgemisch nach Entfernen von Feststoffen aus dem Reaktionsgemisch durch Filtration isoliert wird.

5. Verfahren nach Ansprüchen 1-4, weiterhin umfassend einen Schritt des Unterziehens der Verbindung der Formel (3) (einem) nächsten synthetischen Schritt(en), um letztendlich Everolimus der Formel (1) zu bilden.

6. Verfahren nach Anspruch 5, wobei der synthetische Schritt auf die Gegenwart der Verbindung der Formel (3) durch ein Derivatisierungsverfahren überprüft wird, das auf der Reaktion der Verbindung (3) mit einem Derivatisierungsmittel basiert.

7. Verfahren nach Ansprüchen 6, wobei das Derivatisierungsmittel ein sekundäres aromatisches Amin ist.

8. Verfahren nach Ansprüchen 6-7, wobei das Derivatisierungsmittel N-Methylanilin ist.

9. Verfahren zur Stabilisierung der Verbindung der allgemeinen Formel (3), 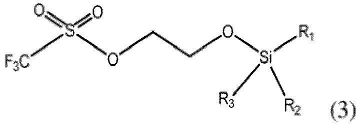 wobei R1, R2, R3 unabhängig eine gerade oder verzweigte Alkylgruppe von 1 bis 10 Kohlenstoffatomen und/oder eine Phenylgruppe sind,
welches umfasst
a) Umsetzen von 2-([Trisubstituiertem]silyloxy)ethanol der allgemeinen Formel (5): 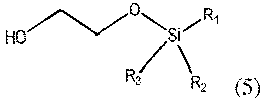 mit Trifluormethansulfonsäureanhydrid in der Gegenwart einer Base, wobei die Base aus N-Ethyl-N-isopropyl-2-aminopropan oder Triisobutylamin ausgewählt ist, in einem Alkanlösungsmittel mit 5 bis 12 Kohlenstoffen, wie n-Pentan, n-Hexan oder n-Heptan, wobei "Alkan" eine gerades oder verzweigtes oder cyclisches Alkan sowie Gemische davon bedeutet, einschließlich Gemischen davon mit einem Aren, z.B. Benzol oder Toluol, um 2-([Trisubstituiertes]silyloxy)ethyltriflat der allgemeinen Formel (3) zu bilden; und
b) Entfernen der gebildeten Triflatsalze der Base aus dem Reaktionsgemisch durch Filtration.

## Revendications

1. Un procédé pour préparer le composé de formule générale (3) 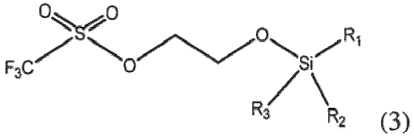 dans laquelle R₁, R₂, R₃ sont, indépendamment les uns des autres, un groupe alkyle linéaire ou ramifié comprenant 1 à 10 atomes de carbone et/ou un groupe phényle, qui comprend
a) la réaction de 2-[silyloxy(trisubstitué)]-éthanol de formule générale (5): 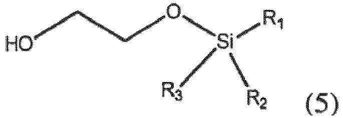
avec l'anhydride trifluorométhane sulfonique en présence d'une base, pour former du triflate de 2-[silyloxy(trisubstitué)]-éthyle de formule générale (3) et un sel d'acide triflique avec la base,
**caractérisé en ce que** la réaction est effectuée dans un solvant alcane comportant de 5 à 12 atomes de carbone, dans lequel par "alcane" on entend un alcane linéaire, ramifié ou cyclique ainsi que leurs mélanges, incluant leurs mélanges avec un arène, par exemple, du benzène ou du toluène et dans lequel la base est sélectionnée parmi le N-éthyl-N-isopropyl-2-aminopropane ou la triisobutylamine,
b) l'élimination par filtration du sel de l'acide triflique avec la base.

2. Le procédé selon la revendication 1, dans lequel l'alcane consiste en n-pentane, n-hexane ou n- heptane.

3. Le procédé selon les revendications 1-2, dans lequel le composé (3) consiste en triflate de 2-(t-hexyldiméthylsilyloxy)-éthyle de formule (3C)

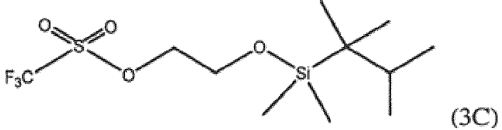

4. Le procédé selon les revendications 1-3, dans lequel le composé de formule (3) est isolé du mélange réactionnel après élimination par filtration des solides contenus dans le mélange réactionnel.

5. Le procédé selon les revendications 1-4 comprenant en outre une étape consistant à soumettre le composé de formule (3) à une nouvelle étape(s) de synthèse pour finalement obtenir de l'éverolimus de formule 1.

6. Le procédé selon la revendication 5, dans lequel l'étape de synthése est contrôlée quant à la présence du composé de formule (3) par une méthode de dérivatisation basée sur la réaction du composé (3) avec un agent de dérivatisation.

7. Le procédé selon la revendications 6, dans lequel l'agent de dérivatisation est une aminé aromatique secondaire.

8. Le procédé selon les revendications 6-7, dans lequel l'agent de dérivatisation est la N- méthylaniline.

9. Un procédé de stabilisation du composé de formule générale (3), 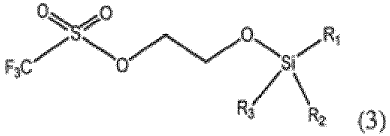 dans laquelle R₁, R₂, R₃ sont, indépendamment les uns des autres, un groupe alkyle linéaire ou ramifié formé de 1 à 10 atomes de carbone et/ou un groupe phényle,
qui comprend :
a) la réaction de 2-[silyloxy (trisubstitué)]-éthanol de formule générale (5): 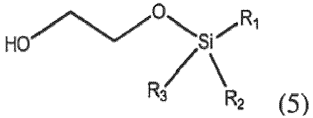 avec l'anhydride trifluorométhane sulfonique en présence d'une base, laquelle base est sélectionnée parmi le N-éthyl-N-isopropyl-2-aminopropane ou la triisobutylamine, dans un solvant alkane comportant de 5 à 12 atomes de carbone, tel que n-pentane, n-hexane ou n-heptane dans lequel par "alcane" on entend un alcane linéaire, ramifié ou cyclique ainsi que leurs mélanges, incluant leurs mélanges avec un arène, par exemple, du benzène ou du toluène pour former du triflate de 2-[silyloxy(trisubstitué)]-éthyle de formule générale (3), et
b) élimination par filtration des sels de triflate de la base formés du mélange réactionnel.
